# EUROPEAN PATENT APPLICATION

(11) **EP 3 445 027 A1**
(43) Date of publication of application: **20.02.2019**
(21) Application number: 17782418.2
(22) Date of filing: 12.04.2017
(51) Int. Cl.: H04M 11/04, A61G 12/00, G06Q 50/22, G08B 21/02, G08B 25/04

(54) **WATCHING SYSTEM AND MANAGEMENT SERVER**

(30) Priority: 14.04.2016 JP 2016080922
(71) Applicant: Konica Minolta, Inc., Tokyo 100-7015 (JP)
(72) Inventor: KAWAZU Keiichi, Tokyo 100-7015 (JP); OKADA Takashi, Tokyo 100-7015 (JP); ATARASHI Yuichi, Tokyo 100-7015 (JP)
(74) Representative: Alton, Andrew
(86) International application number: PCT/JP2017/014923
(87) International publication number: WO 2017/179605

(57) **Abstract**

Provided are a watching system and a management server that allow recovery to be performed easily in interruption of communication between a mobile terminal and a management server, while reducing the burden of a caregiver, for example. In the watching system; when the mobile terminal transmits, to the management server through a network, login information including identifier information of the mobile terminal and information regarding a user operating the mobile terminal, the management server permits the mobile terminal to log in and the permission enables information to be downloaded from the management server to the mobile terminal; when the mobile terminal transmits logout information to the management server, the management server permits the mobile terminal to log out and the permission disables the information from being downloaded from the management server to the mobile terminal; an information saving unit that saves the login information used for the login of the mobile terminal, is further provided; when the communication is unavailable between the mobile terminal and the management server before the transmission of the logout information after the mobile terminal transmits the login information, a re-login request is automatically transmitted from the mobile terminal and the management server retains the login of the mobile terminal on hold during a standby period; and when the management server receives the re-login request during the standby period after recovery to the communication available between the mobile terminal and the management server, the management server resumes the login to the mobile terminal that has transmitted the re-login request, based on the login information read from the information saving unit.

## Description

### Technical Field

The present invention relates to a watching system and a management server that are to be preferably used in, for example, a care home.

### Background Art

In our country, the low birthrate with longevity has been rapidly progressing due to a growth in the average life span of citizens and a drop in the rate of birth. Alongside this, for example, hospitals and welfare facilities for the aged that accommodate persons requiring care (persons to be cared), have been increasing in number, resulting in a serious situation in which the manpower of nurses and caregivers is chronically insufficient because of heavy working conditions, for example. In particular, at night time, a small number of persons must watch a large number of persons to be cared and thus the burden of a caregiver or the like increases, causing further insufficient manpower.

In order to reduce the burden of the caregiver or the like, a technique of assisting care work is required. In one example, in order to cause a machine to do part of work of a caregiver or the like, various techniques of monitoring a person to be cared have been developed.

Patent Literature 1 discloses a technique of remotely monitoring the current condition of an object to be monitored, the technique including: capturing an image of the object to be monitored, by a camera included in a monitoring device when an abnormality detection device detects an abnormality of the object to be monitored in a facility; and transmitting image data thereof to an external terminal through, for example, a net.

### Citation List

### Patent Literature

Patent Literature 1: JP 2005-253025 A

### Summary of Invention

### Technical Problem

The use of the monitoring device in Patent Literature 1 with a person to be cared set as an object to be monitored, enables unmanned monitoring thereto, so that the burden of a caregiver or the like is reduced. In a case where the caregiver or the like is notified of an abnormality of the person to be cared, an alarm can be displayed on a stationary terminal installed in, for example, a nurse station. However, for example, at night time, the caregiver or the like does not necessarily stay in the nurse station and there is a case where the caregiver or the like is out for watching. Thus, there is a risk that noticing the abnormality of the person to be cared is late. In contrast to this, if information indicating occurrence of the abnormality of the person to be cared is transmitted to a mobile terminal always carried by the caregiver or the like and then an alarm is displayed on the basis thereof, a prompt action can be expected even while the caregiver or the like is watching. Meanwhile, because information on the person to be cared is related to privacy, it is improper that anyone is allowed to access the information. Thus, during the use of the mobile terminal, preferably, the caregiver directly transmits login information to a management server and acquires login authentication such that necessary information can be acquired.

The mobile terminal can perform communication only within a certain spatial range (within a communication service area) around an access point of a local area network (LAN), but, for example, in a wide facility, there is a location out of the communication service area even in the facility. Thus, once the caregiver or the like goes out of the communication service range, the communication between the mobile terminal and the management server is interrupted. When the management server forcibly performs logout processing in response to the interruption, the necessary information cannot be acquired from the management server even when the caregiver or the like returns within the communication service area, again. In order to acquire the information, inconveniently, the caregiver or the like operates singly and transmits the login information to the management server afresh, so as to acquire the login authentication again.

In addition to the case where the mobile terminal goes out of the communication service area, a similar problem can occur, for example, in a case where the mobile terminal in a login falls accidentally, a battery comes off, and instantaneous interruption of the system occurs, or in a case where temporary trouble occurs in the system due to a local power failure.

The present invention has been made in consideration of the circumstances, and an object of the present invention is to provide a watching system and a management server that allow recovery to be performed easily in interruption of communication between a mobile terminal and the management server, while reducing the burden of a caregiver, for example.

### Solution to Problem

In order to achieve at least one in the object described above, a watching system according to one aspect of the present invention, includes:
a sensor device configured to input data from a person to be watched, the sensor device being configured to output a signal corresponding to the data;
a management server communicably connected to the sensor device through a network; and
a mobile terminal capable of wirelessly communicating with the management server through the network, the mobile terminal being configured to output a watching alarm, based on the signal transmitted from the sensor device through the management server,
in which, when the mobile terminal transmits, to the management server through the network, login information including identifier information of the mobile terminal and information regarding a user operating the mobile terminal, the management server permits the mobile terminal to log in and the permission enables information to be downloaded from the management server to the mobile terminal,
when the mobile terminal transmits logout information to the management server, the management server permits the mobile terminal to log out and the permission disables the information from being downloaded from the management server to the mobile terminal,
an information saving unit that saves the login information used for the login of the mobile terminal, is further provided,
when the communication is unavailable between the mobile terminal and the management server before the transmission of the logout information after the mobile terminal transmits the login information, a re-login request is automatically transmitted from the mobile terminal and the management server retains the login of the mobile terminal on hold during a standby period, and
when the management server receives the re-login request during the standby period after recovery to the communication available between the mobile terminal and the management server, the management server resumes the login to the mobile terminal that has transmitted the re-login request, based on the login information read from the information saving unit.

In order to achieve at least one in the object described above, a management server according to one aspect of the present invention, includes:
a communication unit configured to communicate with a plurality of sensor devices and a plurality of mobile terminals through a network, the plurality of sensor devices each configured to input data from a person to be watched, each being configured to output a signal corresponding to the data, the plurality of mobile terminals being configured to output watching alarms, based on the signals transmitted to the sensor devices;
a storage unit configured to store a table including the plurality of sensor devices associated with the plurality of mobile terminals;
a notification processing unit configured to determine whether each of the plurality of mobile terminals is in a login, the notification processing unit being configured to issue, when receiving data from one sensor device of the plurality of sensor devices, notification to a mobile terminal in the login from mobile terminals associated with the one sensor device, the mobile terminals being stored in the storage unit; and
an information saving unit configured to save login information used for the login of the mobile terminal,
in which, when the communication unit receives the login information including identifier information of the mobile terminal and information regarding a user operating the mobile terminal, transmitted from the mobile terminal through the network, the mobile terminal is permitted to log in and the permission enables information to be downloaded to the mobile terminal,
when the communication unit receives logout information transmitted from the mobile terminal, the mobile terminal is permitted to log out and the permission disables the information from being downloaded to the mobile terminal,
when the communication is unavailable between the management server and the mobile terminal before the transmission of the logout information after the mobile terminal transmits the login information, a re-login request is automatically transmitted from the mobile terminal and the login of the mobile terminal is on hold during a standby period of the management server, and
when the communication unit receives the re-login request during the standby period after recovery to the communication available between the management server and the mobile terminal, the login is resumed to the mobile terminal that has transmitted the re-login request, based on the login information read from the information saving unit.

### Advantageous Effects of Invention

According to the present invention, a watching system and a management server can be provided that allow recovery to be performed easily in interruption of communication between a mobile terminal and the management server, while reducing the burden of a caregiver, for example.

### Brief Description of Drawings

Fig. 1 is a diagram illustrating the entire configuration of a watching system in the present embodiment.
Fig. 2 is a diagram illustrating the configuration of each sensor box in the watching system according to the present embodiment.
Fig. 3 is a diagram illustrating the configuration of a management server in the watching system according to the present embodiment.
Fig. 4 is a diagram illustrating the configuration of a mobile terminal in the watching system according to the present embodiment.
Fig. 5 is a table illustrating the configuration of a destination information table in the watching system according to the present embodiment.
Fig. 6 is a ladder-type flowchart illustrating the operation in the watching system according to the present embodiment.
Fig. 7 is a diagram illustrating exemplary display screens of the mobile terminal according to the present embodiment.
Fig. 8 is a diagram illustrating an exemplary display screen of the mobile terminal, according to a modification.
Fig. 9 is a ladder-type flowchart of communication processing to be performed between a mobile terminal TA and the management server SV.
Fig. 10 is an exemplary login screen display of the mobile terminal.
Fig. 11 is a table illustrating a login list stored in an SV storage unit 22 of the management server SV.
Fig. 12 is an exemplary screen display of the mobile terminal, indicating that no communication is available.
Fig. 13 is a ladder-type flowchart of login processing to be performed between the mobile terminal TA and the management server SV according to a different embodiment.

### Description of Embodiments

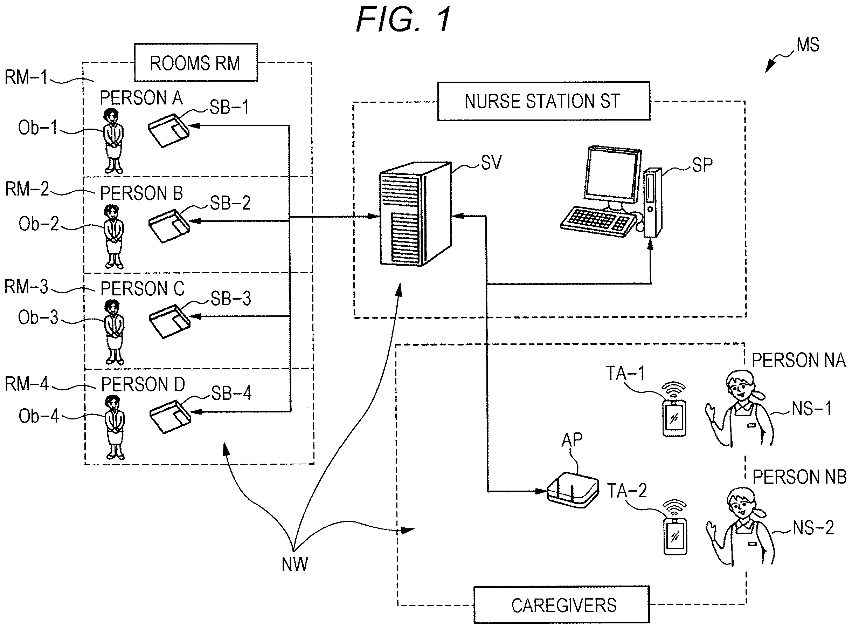

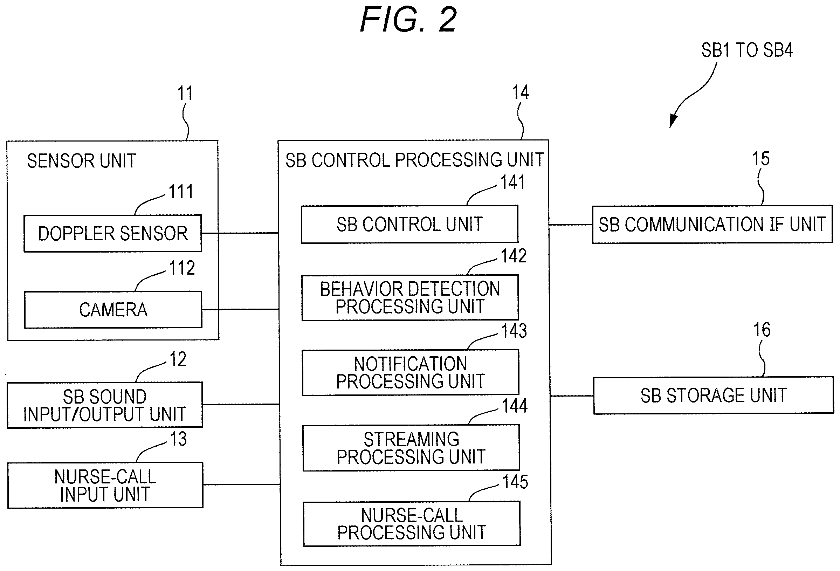

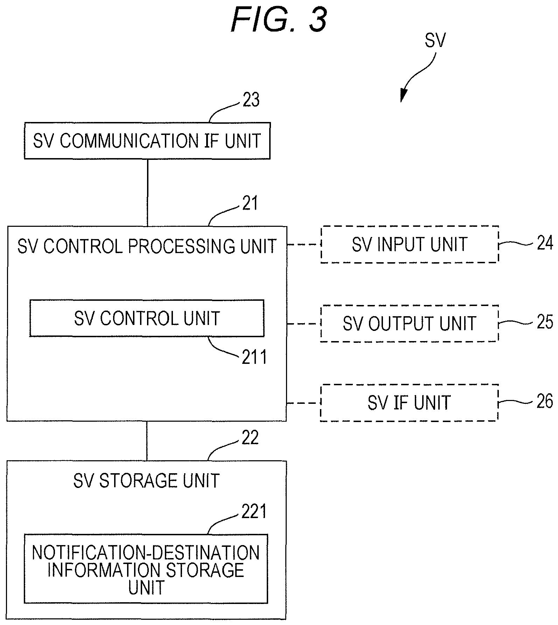

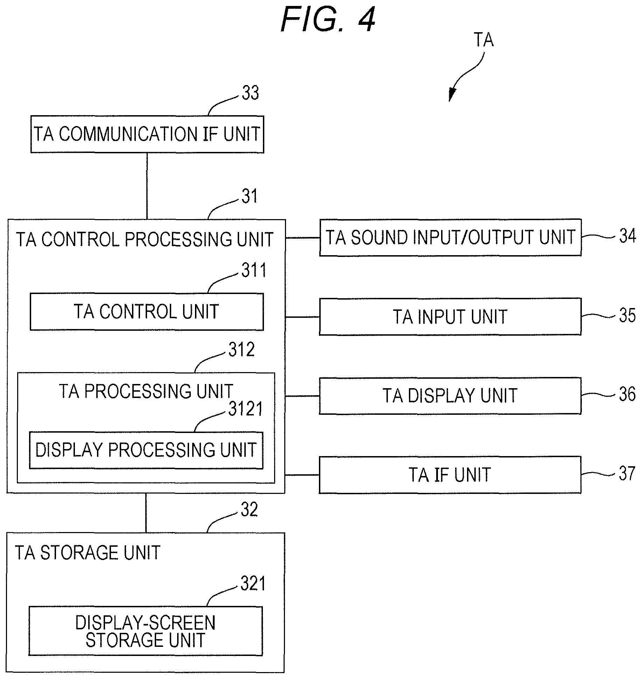

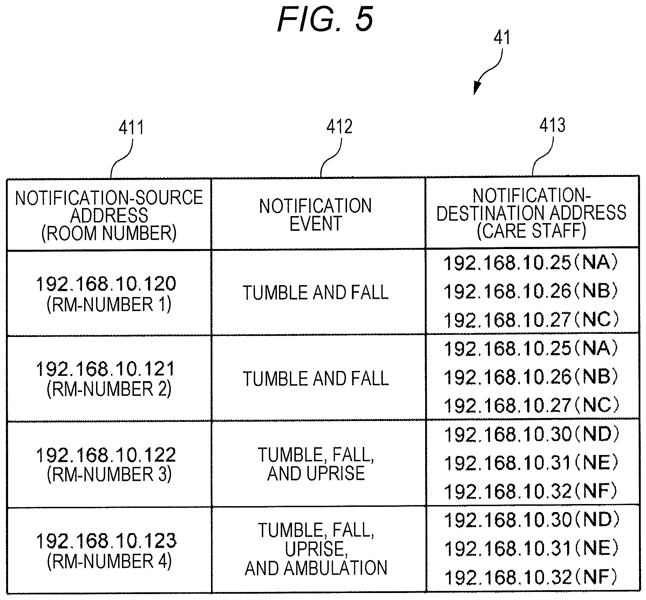

Embodiments according to the present invention will be described below on the basis of the drawings. Fig. 1 is a diagram illustrating the entire configuration of a watching system in the present embodiment. Fig. 2 is a diagram illustrating the configuration of each sensor box in the watching system according to the present embodiment. Fig. 3 is a diagram illustrating the configuration of a management server in the watching system according to the present embodiment. Fig. 4 is a diagram illustrating the configuration of a mobile terminal in the watching system according to the present embodiment. Fig. 5 is a table illustrating the configuration of a destination information table in the watching system according to the present embodiment. In the present specification, "care" is a concept including "nursing".

The watching system in the present embodiment, has a function of detecting the conditions of persons to be monitored (hereinafter, referred to as object persons) Ob who are objects to be watched, by sensor boxes SB corresponding thereto, watching the object persons Ob, and assisting care.

For example, as illustrated in Fig. 1, the watching system MS has: the sensor boxes SB (SB-1 to SB-4) installed in the rooms of the object persons; a management server SV; a stationary terminal device SP; and mobile terminals TA (TA-1 and TA-2) managed by caregivers, each being connected communicably by wireless or by wire through a network (networks and communication lines) NW including a local area network (LAN), a telephone network, and a data communication network. The network NW may include a relay, such as a repeater, a bridge, a router, or a cross-connect, that relays a communication signal. In the example illustrated in Fig. 1, the plurality of sensor boxes SB-1 to SB-4, the management server SV, the stationary terminal device SP, and the plurality of mobile terminals TA-1 and TA-2 are communicably connected to each other in the wireless LAN (e.g., a LAN compliant with the IEEE802.11 standard) NW including an access point AP. The sensor boxes SB each correspond to an exemplary sensor device, and the stationary terminal device SP and the mobile terminals TA each correspond to an exemplary terminal.

The object persons Ob each are, for example, a person requiring nursing due to a disease or an injury, a person requiring care due to deterioration in physical ability, or a person who lives alone. In particular, from the viewpoint of early discovery and early treatment, the object persons Ob each are preferably a person who requires, in a case where a predetermined disadvantageous event, such as an abnormal condition, occurs to the person, discovery thereof. Thus, in accordance with the types of the object persons Ob, the watching system MS is preferably provided in a building, such as a hospital, a welfare facility for the aged, or a dwelling unit. In the example illustrated in FIG. 1, the watching system MS is provided in the building of a care home including a plurality of rooms, such as the plurality of rooms RM occupied by the plurality of object persons Ob and a nurse station ST.

First, the sensor boxes SB will be described. The sensor boxes SB disposed correspondingly on, for example, the ceilings or walls in the rooms RM of the object persons Ob, each have a communication function of communicating with, for example, the management server SV through the network NW. More specifically, as illustrated in Fig. 2, the sensor boxes SB each include a sensor unit 11, an SB sound input/output unit 12, a nurse-call input unit 13, an SB control processing unit 14, an SB communication IF unit 15, and an SB storage unit 16.

The sensor unit 11 connected to the SB control processing unit 14, includes a device that detects the condition of the object person Ob in accordance with the control of the SB control processing unit 14 and outputs condition data corresponding thereto. In the present embodiment, examples of the condition of the object person Ob include an uprise, an ambulation, a tumble, a fall, and an abnormality in slight body movement of the object person Ob, and thus the sensor unit 11 includes, for example, a Doppler sensor 111 and a camera 112, in order to detect the examples.

The Doppler sensor 111 includes a body-movement sensor that transmits a transmission wave, receives the reflected wave of the transmission wave reflected from an object, and outputs a Doppler signal having a Doppler frequency component on the basis of the transmission wave and the reflected wave. In a case where the object is moving, because the frequency of the reflected wave shifts, due to the so-called Doppler effect, in proportion to the speed of movement of the object, a difference (Doppler frequency component) occurs between the frequency of the transmission wave and the frequency of the reflected wave. The Doppler sensor 111 generates, as the Doppler signal, a signal having the Doppler frequency component, and outputs the signal to the SB control processing unit 14. The transmission wave that may be, for example, an ultrasonic wave or a microwave, is the microwave in the present embodiment. The microwave can reflect on the body surface of the object person Ob after penetrating through the clothes, and thus can preferably detect movement of the body surface even when the object person Ob is wearing clothing.

The camera 112 connected to the SB control processing unit 14 includes a device that generates an image (image data) in accordance with the control of the SB control processing unit 14. The camera 112 is disposed such that the camera 112 can monitor a space in which the object person Ob to be monitored intends to stay (space in the room RM in the example illustrated in Fig. 1). The camera 112 captures, as an object to be captured, the space from the above, generates a bird's eye image (image data) of the object to be captured, and outputs the image of the object to be captured to the SB control processing unit 14. Preferably, because the probability is high that the entire object person Ob can be captured, the camera 112 is provided such that the object to be captured can be captured from right above a previously set head intended position (typically, the provision position of a pillow) at which the head of the object person Ob is intended to be located, on bedding on which the object person Ob lies (e.g., a bed). The sensor boxes SB each acquire an image of the object person Ob captured from above the object person Ob, preferably, an image captured from right above the head intended position, by the camera 112 of the sensor unit 11.

Although the camera 112 may include a device that generates a visible-light image, in the present embodiment, the camera 112 has a function of generating an infrared image such that the object person Ob can be monitored relatively in the dark. For example, in the present embodiment, the camera 112 includes a digital infrared camera including: an image-formation optical system that forms an infrared optical image of the object to be captured, onto a predetermined image-formation face; an image sensor disposed such that a light-receiving face agrees with the image-formation face, the image sensor being to convert the infrared optical image of the object to be captured into an electric signal; and an image processing unit that performs image processing to an output of the image sensor, to generate image data that is data indicating an infrared image of the object to be captured. In the present embodiment, the image-formation optical system of the camera 112 preferably includes a wide-angle optical system having an angle of view at which the entire room RM of the object person Ob can be captured (a so-called wide-angle lens (a fisheye lens is included)). An infrared illuminating device that illuminates the space with infrared rays, may be further provided.

The SB sound input/output unit 12 connected to the SB control processing unit 14 includes a circuit that acquires external sound and inputs the external sound into the sensor box SB. Additionally, the SB sound input/output unit 12 generates and outputs sound corresponding to an electric signal indicating sound, in accordance with the control of the SB control processing unit 14. For example, the SB sound input/output unit 12 includes: a microphone that converts the acoustic oscillation of sound into an electric signal; and a speaker that converts an electric signal of sound into the acoustic oscillation of the sound. The SB sound input/output unit 12 outputs an electric signal indicating external sound to the SB control processing unit 14, and additionally converts an electric signal input from the SB control processing unit 14 into the acoustic oscillation of sound and outputs the acoustic oscillation of sound. Note that, preferably, the SB sound input/output unit 12 is detachable from the sensor box SB, is connected to the SB control processing unit 14 by wireless or by wire, and is disposed at any location in the room. Furthermore, in a case where the SB sound input/output unit 12 is connected to the SB control processing unit 14 by wireless, a device that monitors a remaining battery level in the SB sound input/output unit 12 and notifies an operator or the like of the remaining battery, is preferably provided. Alternatively, in a case where the SB sound input/output unit 12 is connected to the SB control processing unit 14 by wire, a device that notifies the operator or the like of whether proper connection is being performed, is preferably provided.

The nurse-call input unit 13 includes, for example, a push-button switch connected to the SB control processing unit 14. In a case where the resident desires to make a nurse-call, an input operation of the nurse-call input unit 13 causes an electric signal indicating the effect that the nurse-call has been received, to be output from the nurse-call input unit 13 to the SB control processing unit 14, so that a signal is transmitted to the mobile terminal TA of a caregiver or the like. In this case, for example, lighting of a lamp attached to the nurse-call input unit 13, preferably indicates that the signal is being transmitted.

The SB communication IF unit 15 connected to the SB control processing unit 14, includes a communication circuit that performs communication in accordance with the control of the SB control processing unit 14. The SB communication IF unit 15 generates a communication signal accommodating data to be transferred input from the SB control processing unit 14, in accordance with a communication protocol used in the network NW of the watching system MS, and then transmits the generated communication signal to, for example, the management SV through the network NW. In addition, the SB communication IF unit 15 receives a communication signal from, for example, the management server SV through the network NW, and extracts data from the received communication signal. Then, the SB communication IF unit 15 converts the extracted data into data in a format that the SB control processing unit 14 can process, and outputs the data to the SB control processing unit 14. Note that the SB communication IF unit 15 may further include an interface circuit that performs data input/output with external equipment, the interface circuit being compliant with a standard, such as a mobile-phone communication network, a WiFi standard, a Bluetooth (registered trademark) standard, an infrared data asscoiation (IrDA) standard, or a universal serial bus (USB) standard.

The SB storage unit 16 connected to the SB control processing unit 14, includes a circuit that stores various programs and various types of data in accordance with the control of the SB control processing unit 14. The various programs include a control processing program, such as a monitoring processing program of performing information processing regarding monitoring for the object person Ob. For example, the monitoring processing program includes: a notification processing program of issuing notification to the outside in a case where a predetermined event occurs; a processing program of distributing an image (moving image) captured by the camera 112 to the stationary terminal device SP or the mobile terminal TA that has requested the image; and a nurse-call processing program of performing voice conversation with the stationary terminal device SP or the mobile terminal TA with the SB sound input/output unit 12. For example, the various types of data include data necessary for executing each program and data necessary for monitoring the object person Ob in addition to data of a moving image captured by the camera 112 and condition data, such as slight-body-movement data, acquired by the Doppler sensor 111. For example, the SB storage unit 16 includes a read only memory (ROM) that is a nonvolatile memory and an electrically erasable programmable read only memory (EEPROM) that is a rewritable nonvolatile memory. For example, the SB storage unit 16 includes a random access memory (RAM) that is a so-called working memory for the SB control processing unit 14 that stores, for example, data generated during the execution of each program.

The SB control processing unit 14 includes a circuit that receives a nurse call, generates a moving image, and detects a predetermined behavior previously set for the object person Ob. For example, the SB control processing unit 14 includes a central processing unit (CPU) and a peripheral circuit thereof. The execution of the control processing program causes the SB control processing unit 14 to functionally have a sensor control unit (SB control unit) 141, a behavior detection processing unit 142, a notification processing unit 143, a streaming processing unit 144, and a nurse-call processing unit 145.

The SB control unit 141 manages the entire control of the sensor box SB.

The behavior detection processing unit 142 detects the predetermined event previously set for the object person Ob, on the basis of an output of the sensor unit 11. In the present embodiment, as described above, the predetermined event includes the uprise, the ambulation, the tumble, the fall, and the abnormality in slight body movement of the object person Ob. The behavior detection processing unit 142 detects, as the event, the uprise, the ambulation, the tumble, the fall, or the abnormality in slight body movement of the object person Ob, on the basis of the output of the sensor unit 11, and issues notification to the notification processing unit 143. The behavior detection processing unit 142 can distinctively detect the uprise, the ambulation, the tumble, the fall, or the abnormality in slight body movement of the object person Ob, on the basis of the output of the sensor unit 11. For example, the behavior detection processing unit 142 extracts a motion region as a body region of the object person Ob, from an image captured by the camera 112 of the sensor unit 11, determines the attitude of the object person Ob (e.g., an upright position, a sitting position, or a lying position) from the aspect ratio of the extracted motion region, detects the position of the detected motion region, and distinctively determines the uprise, the ambulation, the tumble, or the fall, on the basis of the determined attitude and the detected position of the object person Ob. In a case where detecting a transition from a lying attitude from a sitting attitude, the behavior detection processing unit 142 determines the uprise and determines that an event of the uprise (uprise event) has occurred. Furthermore, in a case where detecting a transition from the sitting attitude to an upright attitude from a bed region to the outside, the behavior detection processing unit 142 determines the ambulation and determines that an event of the ambulation (ambulation event) has occurred. In a case where detecting a lying attitude outside a bed region in a transition from the bed region to the outside, the behavior detection processing unit 142 determines the fall and determines that an event of the fall (fall event) has occurred. For example, in a case where detecting a transition from a sitting attitude or an upright attitude to a lying attitude outside the bed region, the behavior detection processing unit 142 determines the tumble and determines that an event of the tumble (tumble event) has occurred. When the Doppler sensor 111 of the sensor unit 11 detects body movement in a breast region (up-and-down movement of the breast region) along with the respiration of the object person Ob and the behavior detection processing unit 142 detects a disorder in cycle of the body movement of the breast region or the amplitude of the body movement of the breast region that is a previously set threshold value or less, the behavior detection processing unit 142 determines the abnormality in slight body movement and determines that an event of the abnormality in slight body movement (abnormality-in-slight-body-movement event) has occurred. The behavior detection processing unit 142 may have a function of making a determination on the basis of stored object person's daily behavior. For example, when behavior different from the stored normal behavior is taken or stored dementia behavior is taken, the behavior detection processing unit 142 may determine that an abnormal event has occurred and may issue notification to the mobile terminal TA as to be described later. Note that the determination criterion of the behavior detection processing unit 142 (determination algorithm) is preferably standardized between all the sensor boxes SB. Thus, the determination criterion is not required to be changed even when the resident is changed, so that no time and labor are required. Note that the determination criterion of the behavior detection processing unit 142 may be allowed to be customized for each sensor box SB.

In a case where the nurse-call input unit 13 receives an input operation, the nurse-call processing unit 145 notifies the notification processing unit 143 of an event, more specifically, an operation of a nurse call (nurse-call event). The nurse-call processing unit 145 enables the voice conversation with the stationary terminal device SP or the mobile terminal TA through the SB communication IF unit 15 and the network NW.

The notification processing unit 143 issues notification to the outside in a case where the nurse-call event of the nurse-call input unit 13 occurs or the predetermined event including the predetermined behavior of the object person Ob detected by the sensor unit 11 (an event of the uprise, the ambulation, the tumble, or the fall in the present embodiment) occurs in the sensor box SB. More specifically, the notification processing unit 143 generates a notification signal including information indicating the classification of an event occurring (the nurse-call event or the predetermined detection event (information identifying the uprise, the ambulation, the tumble, the fall, or the abnormality in slight body movement, namely, information indicating the type of a predetermined condition classified), and identifier information for specifying and identifying the sensor box SB detecting the object person Ob. Then, the notification processing unit 143 transmits the notification signal from the SB communication IF unit 15 to the management server SV through the network NW. If a request is made from the outside (transmission of a condition-data request signal), the notification processing unit 143 generates a communication signal accommodating condition data including, for example, image data, and transmits the communication signal from the SB communication IF unit 15 to the management server SV through the network NW.

Note that, although the following arrangement is not used in the operation of a ladder-type flowchart to be described later, in a case where a request for streaming-video distribution is made from the stationary terminal device SP or the mobile terminal TA through the network NW and the SB communication IF unit 15, the streaming processing unit 144 distributes, in streaming reproduction, a moving image (e.g., a live moving image) generated by the camera 112 of the sensor unit 11, to the stationary terminal device SP or the mobile terminal TA from which the request has been made, through the SB communication IF unit 15 and the network NW.

Fig. 1 illustrates four number of the first to four sensor boxes SB-1 to SB-4 as an example, the first sensor box SB-1 being provided in the room RM-1 of a person A Ob-1 who is one of the object persons Ob, the second sensor box SB-2 being provided in the room RM-2 of a person B Ob-2 who is one of the object persons Ob, the third sensor box SB-3 being provided in the room RM-3 of a person C Ob-3 who is one of the object persons Ob, the fourth sensor box SB-4 being provided in the room RM-4 of a person D Ob-4 who is one of the object persons Ob.

When the sensor boxes SB each operate, the sensor unit 11 outputs an image generated by capturing the located space from above at a predetermined frame rate, and a Doppler signal to the SB control processing unit 14. The behavior detection processing unit 142 of the SB control processing unit 14 detects, at predetermined time intervals, the presence or absence of the predetermined behavior of the object person Ob, on the basis of the image and the Doppler signal. When detecting the predetermined behavior as the detection event, the behavior detection processing unit 142 notifies the notification processing unit 143 of the detection event together with the classification of the detection event. When receiving the notification of the detection event, the notification processing unit 143 transmits a notification signal from the SB communication IF unit 15 to the management server SV.

Note that, although the following arrangement is not used in the operation of a ladder-type flowchart to be described later, when the nurse-call input unit 13 receives an input operation, the nurse-call processing unit 145 notifies the notification processing unit 143 of the reception event of a nurse call. When receiving the notification of the reception event, the notification processing unit 143 transmits a notification signal from the SB communication IF unit 15 to the management server SV. The management server SV notifies the mobile terminal TA carried by a caregiver who is in charge of the room in which the nurse call has occurred, of the occurrence of the nurse call.

Next, the management server SV will be described. In Fig. 1, the management server SV has a communication function of individually communicating with the different sensor boxes SB, the stationary terminal device SP, and the mobile terminals TA through the network NW. The management server SV has a function of providing information to at least one of the stationary terminal device SP and the mobile terminal TA carried by a caregiver who is in charge of the room in which an event has occurred, with reception of notification from the sensor box SB. The management server SV includes an SV control processing unit 21, an SV storage unit 22, and an SV communication IF unit 23, for example, as illustrated in Fig. 3.

The SV communication IF unit 23 connected to the SV control processing unit 21 includes a communication device that performs communication in accordance with the control of the SV control processing unit 21, similarly to the SB communication IF unit 15. The SV communication IF unit 23 generates a communication signal accommodating data to be transferred input from the SV control processing unit 21, in accordance with the communication protocol used in the network NW of the watching system MS, and transmits the generated communication signal to, for example, the sensor box SB through the network NW. The SV communication IF unit 23 receives a communication signal from, for example, the sensor box SB through the network NW, and extracts data from the received communication signal. Then, the SV communication IF unit 23 converts the extracted data into data in a format that the SV control processing unit 21 can process, and outputs the data to the SV control processing unit 21.

The SV storage unit 22 connected to the SV control processing unit 21, includes a circuit that stores various programs and various types of data in accordance with the control of the SV control processing unit 21. The various programs includes a control processing program, such as a server program of providing data corresponding to a request from a client (e.g., the stationary terminal device SP or each mobile terminal TA in the present embodiment) to the client. The various types of data includes necessary data for executing each program described above, such as monitoring information regarding monitoring for each object person Ob and notification-destination information regarding a notification destination of an event, and data necessary for monitoring each object person Ob. The SV storage unit 22 includes, for example, a ROM and an EEPROM, similarly to the SB storage unit 16. For example, the SV storage unit 22 includes a RAM that is a so-called working memory for the SV control processing unit 21 that stores, for example, data generated during the execution of each predetermined program.

The SV storage unit 22 includes a notification-destination information storage unit 221 that stores the notification-destination information. The notification-destination information storage unit 221 stores the notification-destination information regarding a notification destination of an event. In a case where a notification signal is received from a sensor box SB, the notification-destination information indicates a notification destination to be notified of the event accommodated in the received notification signal. For example, in the present embodiment, the notification-destination information indicates the correspondence relationship between the sensor box SB that is the source from which the notification signal has been transmitted, and a mobile terminal TA to which the notification signal received from the sensor box SB is to be transmitted. For example, as illustrated in Fig. 5, the notification-destination information is stored in a table format in the notification-destination information storage unit 221. A notification-destination information table 41 illustrated in Fig. 5, has: a notification-source address field 411 in which the communication address of the sensor box SB that is the source of the notification signal, is registered; a notification-event field 412 including notification-event information, notification being to be issued in a case where the object person is in the event state; and a notification-destination address field 413 in which the communication address of the mobile terminal TA is registered, the communication address of the mobile terminal TA being associated with the communication address of the sensor box SB registered in the notification-source address field 411, the mobile terminal TA being to be notified of the event accommodated in the notification signal received from the sensor box SB having the communication address. The notification-destination information table 41 has a record for each sensor box SB. Note that the communication address of the sensor box SB may be regarded as the identification ID. Although the notification-source address field 411 is not necessarily required to be provided, the number of the room in which the sensor box SB is provided may be registered for the provision. The communication address of the mobile terminal TA may be regarded as terminal identifier information (terminal ID) for specifying and identifying the mobile terminal TA. The name of a monitor, such as the name of the caregiver carrying the mobile terminal TA, may be registered in the notification-destination address field 413. The notification event item (notification event information) in the notification-event field 412, is settable from the management server SV side, on the basis of the object person Ob of the room in which the sensor box SB is installed. The example of Fig. 5 shows that, for the person A and the person B, notification should be issued when an event of the tumble or the fall occurs, for the person C, notification should be issued when an event of the tumble, the fall, or uprise occurs, and, for the person D, notification should be issued when an event of the tumble, the fall, the uprise, or the ambulation occurs. For example, the notification event is desirably distinguished depending on the object persons because of the conditions of the object persons (there is a person who can be determined that no action is required because there is few problem after uprise, or otherwise) and past action information (there is a person who can be determined that no action is required for one-time ambulation because the person has a habit of repeating the ambulation and bedding a few times in the past).

The SV control processing unit 21 includes a circuit that receives notification from the sensor box SB and transfers the received notification to the stationary terminal device SP and the corresponding mobile terminals TA. The SV control processing unit 21 includes, for example, a central processing unit (CPU) and a peripheral circuit thereof.

An SV control unit 211 of the SV control processing unit 21 manages the entire control of the management server SV.

Note that, as illustrated in Fig. 3, as necessary, the management server SV may further include: a server input unit (SV input unit) 24 that inputs, for example, various commands or various types of data; a server output unit (SV output unit) 25 that outputs, for example, the various commands or the various types of data input by the SV input unit 24 or the information regarding monitoring for each object person Ob; and a server interface unit (SV IF unit) 26 that performs data input/output with external equipment, each unit being connected to the SV control processing unit 21. The management server SV can be configured by, for example, a computer having a communication function.

The stationary terminal device SP installed in the nurse station ST, includes equipment having: a communication function of communicating with, for example, the management server SV through the network NW; a display function of displaying predetermined information; and an input function of inputting a predetermined instruction or predetermined data, the equipment being to function as a user interface (UI) of the watching system MS by input of a predetermined instruction or predetermined data to be given to the management server SV or each mobile terminal TA or by display of a detected result or an image acquired by each sensor box SB. The stationary terminal device SP can be configured by, for example, a computer having a communication function. Preferably, the stationary terminal device SP can grasp an in-room condition through the function of the sensor box SB in each room in which the sensor box SB is installed.

Next, the mobile terminals TA will be described. The mobile terminals TA each include equipment having: a communication function of communicating with, for example, the management server SV through the network NW; a display function of displaying predetermined information; an input function of inputting a predetermined instruction or predetermined data; and a conversation function of performing voice conversation, the equipment being to input a predetermined instruction or predetermined data to be given to the management server SV or a sensor box SB, the equipment being to receive notification of the predetermined event in a case where the predetermined event has occurred in the sensor box SB, the equipment being to perform conversation with the sensor box SB, the equipment being to display a moving image generated by the sensor box SB.

In the present embodiment, for example, as illustrated in Fig. 4, the mobile terminals TA each include a TA control processing unit 31, a TA storage unit 32, a TA communication IF unit 33, a TA sound input/output unit 34, a TA input unit 35, a TA display unit 36, and a TA IF unit 37.

The TA sound input/output unit 34 connected to the TA control processing unit 31, includes a device that acquires external sound and inputs the external sound into the mobile terminal TA. Additionally, the TA sound input/output unit 34 generates and outputs sound corresponding to an electric signal indicating sound, in accordance with the control of the TA control processing unit 31. The TA sound input/output unit 34 includes, for example, a microphone that converts acoustic oscillation into an electric signal, and a speaker that converts an electric signal of sound into the acoustic oscillation of the sound, similarly to the SB sound input/output unit 12. The TA sound input/output unit 34 outputs an electric signal indicating the external sound, to the TA control processing unit 31, and additionally converts an electric signal input from the TA control processing unit 31 into the acoustic oscillation of sound and outputs the acoustic oscillation of sound.

The TA input unit 35 connected to the TA control processing unit 31 includes, for example, a device that receives a predetermined operation and inputs the predetermined operation into the mobile terminal TA, the device including a plurality of input switches to which a predetermined function is allocated. For example, the predetermined operation includes various operations necessary for monitoring, such as an input operation of identification ID for a login, a response operation of responding to a reported nurse call, a request operation of a moving image, and an input operation of the effect that an action, such as lifesaving, nursing, or assistance, is intended to be taken to an reported object person Ob (recovery). The TA display unit 36 that is an indication unit for information and is connected to the TA control processing unit 31, includes a device that displays, for example, the contents of the predetermined operation input from the TA input unit 35 or the monitoring information regarding monitoring for an object person Ob monitored by the watching system MS (e.g., an event occurring in the sensor box SB or an image of the object person Ob), in accordance with the control of the TA control processing unit 31, the device including a display device, such as an LCD or an organic EL display. In the present embodiment, the TA input unit 35 and the TA display unit 36 include a touch panel. In this case, the TA input unit 35 includes, for example, a resistance-film pointing device or a capacitive pointing device that detects and inputs an operation position. The touch panel has the pointing device provided on the display screen of the TA display unit 36. The TA display unit 36 displays one or a plurality of input-content candidates that can be input. When a user (monitor), such as a caregiver, touches the display position at which an input content to be desirably input is displayed, the pointing device detects the position and inputs, as an operation input content of the user, the display content displayed at the detected position into the mobile terminal TA.

The TA IF unit 37 connected to the TA control processing unit 31, includes a device that performs data input/output with external equipment in accordance with the control of the TA control processing unit 31, the device including, for example, an interface circuit compliant with a mobile-phone communication network, a WiFi standard, or a Bluetooth (registered trademark) standard, an interface circuit being to perform infrared communication compliant with an IrDA standard, or an interface circuit compliant with a USB standard.

The TA communication IF unit 33 connected to the TA control processing unit 31, includes a communication device that perform communication in accordance with the control of the TA control processing unit 31, similarly to the SB communication IF unit 15. The TA communication IF unit 33 generates a communication signal accommodating data to be transferred input from the TA control processing unit 31, in accordance with the communication protocol used in the network NW of the watching system MS, and transmits the generated communication signal to, for example, the management server SV through the network NW. The TA communication IF unit 33 receives a communication signal from, for example, the management server SV through the network NW, and extracts data from the received communication signal. Then, the TA communication IF unit 33 converts the extracted data into data in a format that the TA control processing unit 31 can process, and outputs the data to the TA control processing unit 31.

The TA storage unit 32 connected to the TA control processing unit 31, includes a circuit that stores various programs and various types of data in accordance with the control of the TA control processing unit 31, similarly to the SB storage unit 16. The various programs include, for example, a display processing program of processing an operation regarding displaying. The various types of data include each piece of data of, for example, screen information to be displayed in the TA display unit 36. The TA storage unit 32 includes, for example, a ROM and an EEPROM. For example, the TA storage unit 32 includes a RAM that is a so-called working memory for the TA control processing unit 31 that stores, for example, data generated during execution of each predetermined program. The TA storage unit 32 functionally has a display-screen storage unit 321 in order to store each piece of information described above.

The display-screen storage unit 321 stores the screen information to be displayed in the TA display unit 36, in accordance with the control of a display processing unit 3121 to be described later in the TA control processing unit 31.

The TA control processing unit 31 includes a circuit that processes information. The TA control processing unit 31 includes, for example, a CPU and a peripheral circuit thereof, similarly to the SB control processing unit 14. Execution of a control processing program causes the TA control processing unit 31 to functionally have a TA control unit 311 and a TA processing unit 312, and the TA processing unit 312 functionally has the display processing unit 3121.

The TA control unit 311 manages the entire control of the mobile terminal TA.

The display processing unit 3121 processes an operation regarding the displaying of the TA display unit 36. More specifically, the display processing unit 3121 displays a moving image on the TA display unit 36 as necessary.

The mobile terminals TA can be each configured by a portable communication terminal device, such as a so-called tablet computer, a smartphone, or a mobile phone.

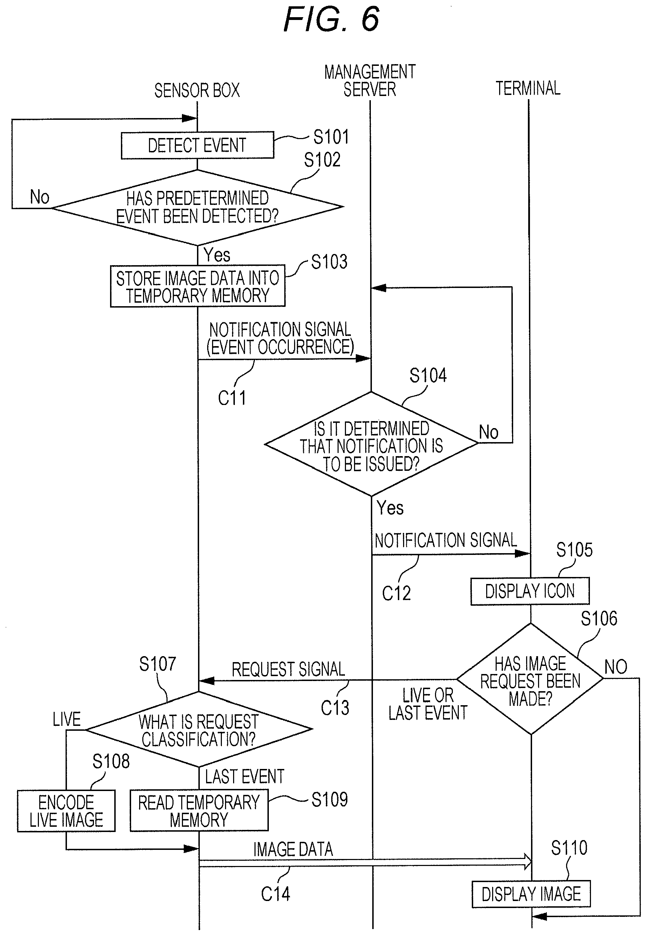

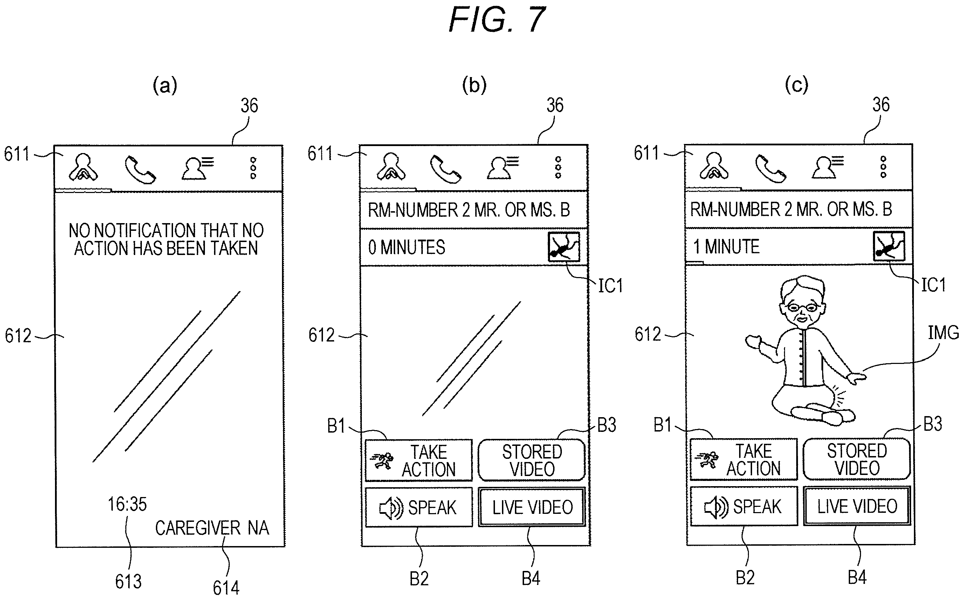

Next, the operation of the watching system according to the present embodiment will be described. Fig. 6 is a ladder-type flowchart illustrating the operation in the watching system according to the present embodiment. Fig. 7 is a diagram illustrating exemplary display screens of each mobile terminal according to the present embodiment.

When the watching system MS having the configuration is activated, the SB control processing unit 14 in each sensor box SB functionally has, due to execution of the control processing program thereof, the SB control unit 141, the behavior detection processing unit 142, the notification processing unit 143, the streaming processing unit 144, and the nurse-call processing unit 145, the SV control processing unit 21 in the management server SV functionally has, due to execution of the control processing program thereof, the SV control unit 211, and the TA control processing unit 31 in each mobile terminal TA functionally has, due to execution of the control processing program thereof, the TA control unit 311 and the TA processing unit 312 functionally having the display processing unit 3121.

The operation of the watching system MS will be described, but the nurse-call event will be omitted. When a mobile terminal TA receives a login operation of a caregiver or the like (the details thereof will be described later), the TA processing unit 312 in the mobile terminal TA causes a standby screen for awaiting a communication signal to the host device, to be displayed on the TA display unit 36. For example, as illustrated in Fig. 7(a), the standby screen includes: a menu-bar region 611 displaying a menu bar; and a standby main region 612 displaying a message indicating on-standby (e.g., "no notification that no action has been taken"), the date and current time, and the user name (caregiver) in a login to the mobile terminal TA. When the standby screen is displayed, the TA control unit 311 causes the mobile terminal TA to wait until the TA communication IF unit 33 receives the communication signal.

At step S101 of Fig. 6, in a sensor box SB, the SB control unit 141 samples output data of the Doppler sensor 111 of the sensor unit 11 in a sampling cycle corresponding to the predetermined frame rate, acquires image data from the camera 112, and then stores the output data and the image data into a ring buffer not illustrated. The behavior detection processing unit 142 analyzes the sampled output data of the Doppler sensor 111 and the acquired image data of the camera 112. As a result of the analysis, at step S102, in a case where the SB control unit 141 detects no predetermined event (the uprise, the ambulation, the tumble, the fall, or the abnormality in slight body movement), the flow goes back to step S101 and the detection operation continues. Meanwhile, when the SB control unit 141 detects the predetermined event, at step S103, at least either the image data or the slight-body-movement data before and after the event, is cut out from the ring buffer and then is stored into the SB storage unit 16. Note that, for example, the detection operation of the sensor box SB can be interrupted only in a set time zone through the stationary terminal SP. In addition, for example, a program in the sensor box SB can be changed through the stationary terminal SP.

Furthermore, the notification processing unit 143 in the sensor box SB transmits a notification signal including event information indicating what type of notification event has occurred and a communication address, to the management server SV through the network NW (C11). Because the notification signal includes no image data of the object person, the information amount thereof is small.

When the notification signal is transmitted from the sensor box SB, at step S104, the management server SV collates the information included in the notification signal, with the notification-destination information table 41 stored in the notification-destination information storage unit 221, and determines, from the communication address of a destination corresponding to the communication address of the source, who the resident is (here, the person B). Furthermore, the management server SV determines whether the type of the notification event (here, the tumble or the fall) corresponding to the resident (the person B) is applicable from the event information in the notification signal. Then, in a case where the event information in the received notification signal indicates the notification event, it is determined that notification is to be issued to a terminal. Meanwhile, in a case where the event information in the received notification signal indicates no notification event (for the person B, the tumble or the fall), it is determined that no notification is to be issued to a terminal, and the management server SV waits the next notification signal.

In a case where determining that notification is to be issued to a terminal, the management server SV collates the notification-destination information table 41 stored in the notification-destination information storage unit 221, and selects, from the communication addresses of the destinations corresponding to the communication address of the source, the mobile terminal TA of a destination. More specifically, the management server SV extracts the communication address of a notification destination registered in the notification-destination address field 413 in the record in which the communication address of the source of the received notification signal is registered in the notification-source address field 411, in the notification-destination information table 41 stored in the notification-destination information storage unit 221. For example, in a case where a notification signal is received from the sensor box SB-2 having the communication address "192.168.10.121", provided in the RM-Number 2, the communication addresses "192.168.10.25", "192.168.10.26", and "192.168.10.27" of three notification destinations registered in the notification-destination address field 413 in the second-line record in which the communication address "192.168.10.121" of the source of the notification signal is registered, are extracted. Note that, in the example, the communication address "192.168.10.25" is a given communication address to the mobile terminal TA-1 carried by a caregiver NA, the communication address "192.168.10.26" is a given communication address to the mobile terminal TA-2 carried by a caregiver NB, and the communication address "192.168.10.27" is a given communication address to a mobile terminal TA-3 carried by a caregiver NC omitted in the figure.

The management server SV transmits a notification signal including information that the tumble or the fall has occurred in the RM-Number 2 (an event signal, namely, a watching alarm), to the selected mobile terminals TA-1 to TA-3 and the stationary terminal SP (C12).

When the management server SV transmits the notification signal, the TA processing unit 312 in the mobile terminal TA switches the screen display of the TA display unit 36 to, for example, that illustrated in Fig. 7(b). In this case, for example, sound or voice may be output (e.g., a voice message of "the fall has occurred in the RM-Number 2") through the TA sound input/output unit 34 serving as an indication unit, or, for example, vibration may notify the caregiver of the reception of the notification signal.

In the example illustrated in Fig. 7(b), the name of the room in which the event has occurred (RM-Number 2, namely, information regarding the sensor box that has transmitted the notification), the name of the resident of the room (Mr. or Ms. B), an icon IC1 indicating the tumble or the fall (namely, information indicating that the event signal (notification) has been received), and the time difference between notification time and the current time (0 minutes) are displayed in the main region 612 (step S105). Simultaneously, buttons B1 (take an action), B2 (speak), B3 (view stored video), and B4 (view live video) are displayed in the main region 612.

Here, when the caregiver touches the button B3 or B4, the TA input unit 35 of the mobile terminal TA reacts thereto. At step S106, the TA control unit 311 determines that an image request has been made, and a request signal (condition-data request signal) is transmitted from the TA communication IF unit to the management server SV through the network NW (C13). Here, the request signal includes a request classification (stored video or live video). The management server SV relays and transmits the request signal to the sensor box SB that is a target. Note that the request signal may be directly transmitted to the sensor box SB.

At step S107, the sensor box SB that has received the request signal, identifies the request classification in the request signal. Because the mobile terminal TA outputs the request signal requiring the stored image in a case where the caregiver touches the button B3, the sensor box SB that has received the request signal through the management server SV, determines that the stored video is being required. At step S109, the image data is read from the SB storage unit 16 and then is transmitted from the SB communication IF unit 15 to the management server SV through the network NW (C14). Alternatively, because the mobile terminal TA outputs the request signal requiring the live image in a case where the caregiver touches the button B4, the sensor box SB that has received the request signal through the management server SV, determines that the live video is being required. At step S108, the live video captured by the camera 112 is encoded and then is transmitted from the SB communication IF unit 15 to the management server SV through the network NW (C14). The live video may be cut out from the ring buffer. The management server SV relays and transmits the image data to the mobile terminal TA that has transmitted the request signal. Note that the image data may be directly transmitted to the mobile terminal TA.

The TA processing unit 312 in the mobile terminal TA that has received the image data, switches the screen display of the TA display unit 36 to, for example, that illustrated in Fig. 7(c). In the example illustrated in Fig. 7(c) in which the stored video has been requested, an image (video) IMG of the person B before and after the tumble in the RM-Number 2 is displayed in the main region 612, and thus the caregiver who has viewed the image IMG can determine whether a prompt action is to be taken for the condition. In a case where the determination cannot be made even with the viewing of the image IMG, a touch of the caregiver on the button B2 enables direct conversation between the person B and the caregiver through the SB sound input/output unit 12 of the sensor box SB, so that the situation can be accurately determined. In a case where the live video has been required, the image displayed on the mobile terminal TA includes the current streaming image. In the example described above, the notification signal is transmitted to the mobile terminal TA, but the notification signal may be transmitted to at least one of the mobile terminal TA and the stationary terminal SP. Note that the image request can be made at any time regardless of the notification signal.

Note that, when the caregiver who has viewed the icon IC1 displayed at step SI05 is located at a place near the RM-Number 2 having the event occurring, the caregiver can rush immediately without viewing the image. Thus, in that case, a touch on the button B1 causes information regarding the effect that the caregiver is to take an action directly, to be transmitted to the stationary terminal SP. Preferably, the touch on the button B1 causes the display corresponding to the notification signal, to be canceled and the display of Fig. 7(a) to return or "a caregiver in charge is taking an action" to be displayed, in at least one of another mobile terminal TA and the stationary terminal SP to which the notification signal have been transmitted.

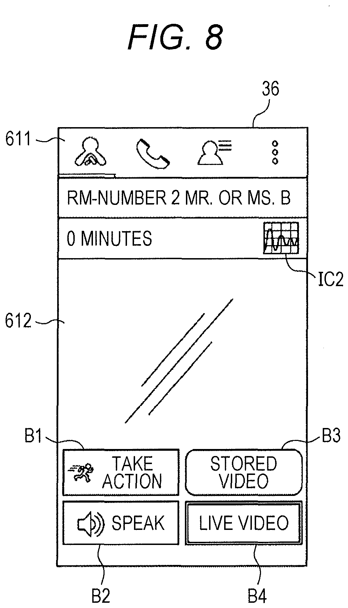

Fig. 8 is a diagram illustrating an exemplary different display screen of each mobile terminal. In the example described above, the sensor box SB determines that the object person has tumbled down or fell down, from the acquired image data, and transmits the notification signal to the management server SV. However, for example, an abnormality in slight body movement can be detected and a notification signal can be transmitted to the management server SV. In that case, similarly to the example described above, the notification signal indicating the abnormality in slight body movement may be transmitted from the management server SV to a selected mobile terminal TA, and an icon IC2 indicating the abnormality in slight body movement, may be displayed on the mobile terminal TA that has received the notification signal. In this case, a touch of the caregiver on the button B3 enables a request for a transfer of an image in the room (or a transfer of slight-body-movement data) and a touch of the caregiver on the button B2 enables direct conversation between the object person and the caregiver through the SB sound input/output unit 12 of the sensor box SB, so that the situation can be accurately determined. Integration of analysis of an image captured by the camera and the condition of slight body movement detected by the Doppler sensor, may be used for event determination.

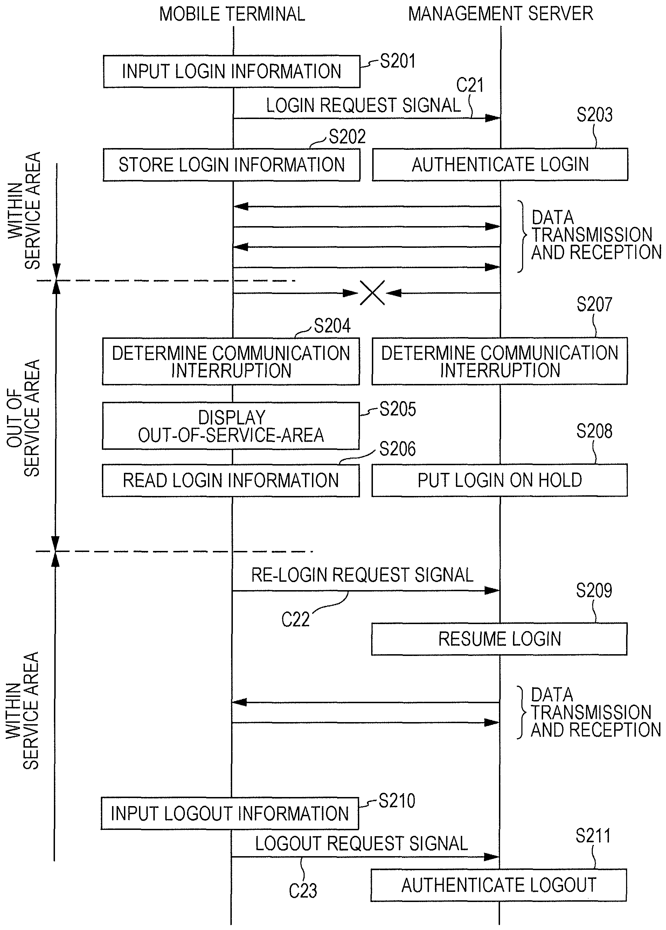

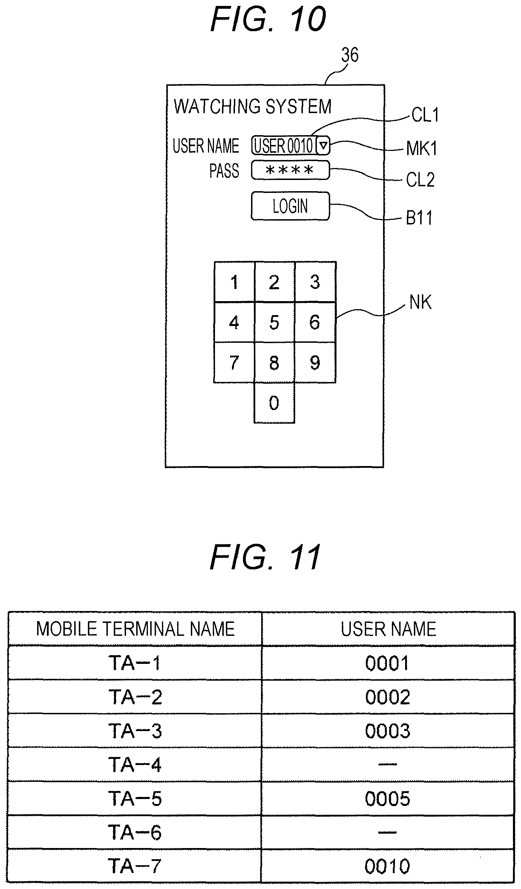

Next, login processing in the management server will be described. Fig. 9 is a ladder-type flowchart of the login processing to be performed between a mobile terminal TA and the management server SV, and illustrates an example in which an information saving unit is located on the mobile terminal TA side. Here, one (user name: 0010) of caregivers who have started working, operates a main switch of the mobile terminal TA managed by the one caregiver, on. Then, a login screen as illustrated in Fig. 10 is displayed. Although a standard login screen is displayed on a mobile terminal using an OS, such as "Android (registered trademark)", here the mobile terminal TA is customized such that the login screen as illustrated in Fig. 10 is displayed initially.

At step S201 of Fig. 9, the user performs a login operation, so that the mobile terminal in a login is effective as part in the watching system. More specifically, when the user taps a triangular mark MK1 at the right end of an input field CL1 for "user name" on the login screen illustrated in Fig. 10, a plurality of alternatives is displayed. The user taps his/her user name "0010" from the plurality of alternatives and selects the user name (user ID). Furthermore, the user inputs, into an input field CL2 for "pass", a four-digit number with a numeric key NK displayed below the input field CL2. After that, the user taps a button B11, so that the login operation is completed. Note that the input of the pass may be omitted.

The login operation at step S201 causes a login request signal including, for example, the user name and the terminal ID (identifier information) as information regarding the user, to be transmitted from the mobile terminal (here, TA-7) to the management server SV (C21). Furthermore, at step S202, the mobile terminal TA-7 stores the user name and the terminal ID that are login information used for the login, into the TA storage unit 32 (preferably, a nonvolatile memory) serving as the information saving unit.

With reference to database stored in the SV storage unit 22, the management server SV that has received the login request signal from the mobile terminal TA-7, verifies whether the user name and the pass are in agreement and performs login authentication (step S203). When the user name and the pass are in agreement, the management server SV performs the login processing.

Fig. 11 is a table illustrating a login list stored in the SV storage unit 22 of the management server SV. The login list includes mobile terminals each specified with identification ID, the mobile terminals being in association with users currently in logins with the mobile terminals. For mobile terminals (TA-4 and TA-6) that have not been used, the user-name field is empty. Seven mobile terminals TA-1 to TA-7 can be used in the present embodiment. When the user "0010" logs in from the mobile terminal TA-7, the user "0010" is added to the login list of the Fig. 11. The number of mobile terminals that can be used, is arbitrary.

When the management server SV completes the login authentication, data transmission and reception start between the mobile terminal TA-7 and the management server SV (downloading and uploading due to a request from the mobile terminal TA-7). Thus, because being able to download, for example, information regarding the object person Ob of which the user "0010" is in charge, the user "0010" can perform, for example, proper care.

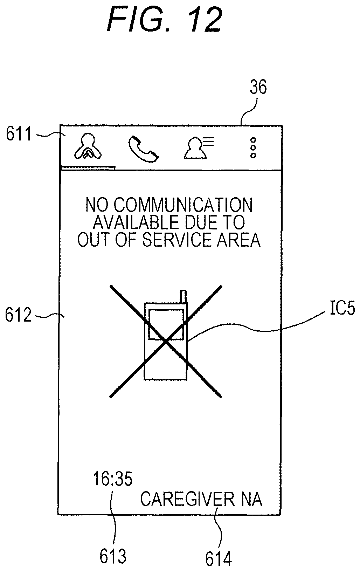

Here, it is assumed that the user "0010" who is carrying the mobile terminal TA-7, moves from a predetermined range around the access point AP (a communicable space) to the outside (out of a service area). This state renders the communication between the mobile terminal TA-7 and the management server SV, unavailable. In this case, because the mobile terminal TA-7 cannot download the information from the management server SV, at step S204, the mobile terminal TA-7 determines that the communication with the management server SV has been interrupted. At step S205, the mobile terminal TA-7 displays a message of "no communication is available due to out of the service area" (information indicating that the communication with the management server is unavailable) together with an icon IC5 indicating that no communication is available, in the main region 612 as illustrated in Fig. 12. The user "0010" who has viewed this, understands currently out of the service area. In this case, because the mobile terminal TA-7 is rendered into a state in which no input is acceptable, keeping displaying the screen illustrated in Fig. 12, any input is rejected. Thus, even when the mobile terminal TA-7 is carried out of the service area by a third party, the privacy of the object person is secured.

At step S206, the mobile terminal TA-7 reads the login information saved in the TA storage unit 32, creates a re-login request signal including the user name and the terminal ID, and automatically sends the re-login request signal through the TA communication IF unit 33. However, as long as the mobile terminal TA-7 is out of the service area, the re-login request signal is not received by the management server SV and no response signal is made. Thus, the mobile terminal TA-7 repeatedly sends the re-login request signal for a predetermined sending time (e.g., one minute from the communication interruption). Note that, in a case where the sending time has elapsed, the screen display of the mobile terminal TA-7 can be returned to the login screen illustrated in Fig. 10.

Meanwhile, once the mobile terminal TA-7 goes out of the service area, no response is made from the mobile terminal TA-7 to the notification from the management server SV and no information can be transmitted. Thus, at step S207, the management server SV determines that the communication with the mobile terminal TA-7 has been interrupted. A self-position detection device may be provided to the mobile terminal TA-7 and it may be supplementarily determined whether the mobile terminal TA-7 is out of the service area. Here, at step S208, the management server SV puts the login on hold without performing logout processing immediately. Specifically, with the mobile terminal TA-7 remaining associated with the user "0010" in the login list of Fig. 11, the management server SV waits until the mobile terminal TA-7 transmits the re-login request, for a standby time (e.g., three minutes from the communication interruption) longer than the sending time. During the login on hold, for example, no information is provided whatsoever from the management server SV to the mobile terminal TA-7 (e.g., there is no case where a notification signal is transmitted from the sensor box in the room of which the user "0010" is in charge). Note that, in a case where there is transmission to the mobile terminal TA-7, the content thereof is stored in the SV storage unit 22. Note that, in a case where the standby time has elapsed, the management server SV can perform the logout processing to the mobile terminal TA-7. The performance of the logout processing causes the user "0010" to be deleted from the login list.

Here, when the user "0010" carrying the mobile terminal TA-7 returns within the predetermined range (within the service area) around the access point AP, the mobile terminal TA-7 can communicate with the network NW through the TA communication IF unit 33. Thus, if during the sending time, the re-login request signal is transmitted to the management server SV (C22).

In response to this, in a case where the management server SV receives the re-login request signal during the standby time, at step S209, on the basis of the user name and the terminal ID included in the re-login request signal, the management server SV determines that the communication with the mobile terminal TA-7 having the login on hold due to the communication interruption, has recovered, and performs login resumption processing. Specifically, the data transmission and reception with the mobile terminal TA-7 are resumed. In addition, if the transmission to the mobile terminal TA-7, performed during the login on hold, has been stored in the SV storage unit 22, the transmission is read and then is transferred to the mobile terminal TA-7. Thus, the mobile terminal TA-7 determines that the communication with the management server SV has been resumed, and changes the screen to, for example, the standby display illustrated in Fig. 8(a). Note that, in the example described above, because the re-login request signal to be transmitted to the management server SV, includes the user name and the terminal ID, new login processing can be performed with the user name and the terminal ID. In that case, although the login list is not necessarily required to be provided, time and labor that the user inputs the login information afresh, can be avoided.

When the user "0010" finishes working, an operation of a logout button (not illustrated) displayed on the mobile terminal TA-7, causes logout information to be input (step S210). Then, a logout request signal including the user name and the terminal ID as the logout information, is transmitted from the mobile terminal TA-7 to the management server SV (C23). When receiving the logout request signal, at step S211, the management server SV performs the logout processing. Specifically, the management server SV deletes, in the login list of Fig. 11, the user name corresponding to the mobile terminal TA-7 and renders the field empty. Until the next login is performed, for example, no information is provided whatsoever from the management server SV to the mobile terminal TA-7.

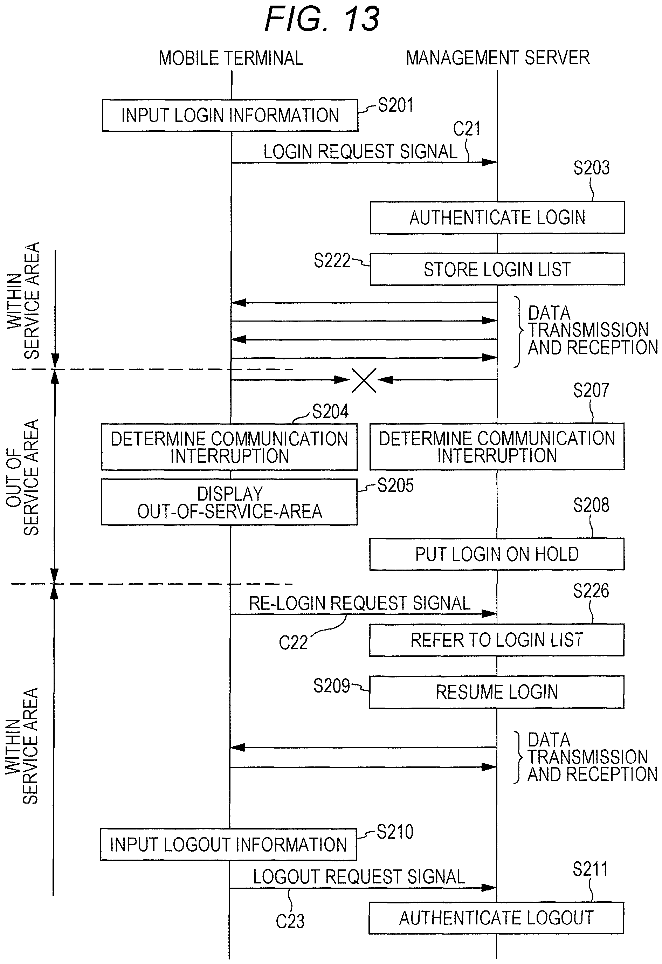

Fig. 13 is a ladder-type flowchart of the login processing to be performed between the mobile terminal TA and the management server SV according to a different embodiment, and illustrates an example in which the information saving unit is located on the management server SV side. Specifically, the mobile terminal TA-7 does not store, for example, the user name and the terminal ID, but, after the login authentication at step S203, the login list storing, for example, the user name in association with the terminal ID (refer to Fig. 11), is created and then is stored in the SV storage unit 22 serving as the information saving unit in the management server SV (step S222).

Furthermore, when the mobile terminal TA-7 goes out of the service area and then returns within the service area again within the sending time, the mobile terminal TA-7 transmits the re-login request signal including the terminal ID (or the user name). Then, when the management server SV receives the re-login request signal, at step S226, the management server SV refers to the login list saved in the SV storage unit 22 for the terminal ID in the re-login request signal. If the terminal ID is present in the login list, the management server SV determines that the communication with the mobile terminal TA-7 having the login on hold due to the communication interruption, has recovered, and performs, at step S209, the login resumption processing. The configuration except for that, is similar to that of the embodiment described above, and thus the description thereof will be omitted.

The present invention is not limited to the embodiments described in the specification, and it is clear to a person skilled in the art that other embodiments and modifications are to be included, from the embodiments and the technical idea described in the present specification. The descriptions and the embodiments in the specification are consistently for the exemplification, and thus the scope of the present invention is indicated in the claims to be described later. For example, the example in which the communication with the management server is unavailable when the mobile terminal goes out of the service area, has been given in each embodiment described above. However, the present invention is not limited to this, and includes, for example, a case where the mobile terminal falls accidentally, a battery comes off, and instantaneous interruption of the system occurs, but the battery is restored immediately and reconnection is made to the network, a case where, for example, a temporary power failure in the home causes the communication through the network, to be unavailable, and a case where occurrence of some kind of failure in the management server itself causes the communication to be unavailable.

### Reference Signs List

- 11: sensor unit
- 12: SB sound input/output unit
- 13: nurse-call input unit
- 14: SB control processing unit
- 15: SB communication IF unit
- 16: SB storage unit
- 21: SV control processing unit
- 22: SV storage unit
- 23: SV communication IF unit
- 24: SV input unit
- 31: SV control processing unit
- 32: SV storage unit
- 33: SV IF unit
- 34: TA sound input/output unit
- 35: TA input unit
- 36: TA display unit
- 37: TA IF unit
- 41: notification-destination information table
- 111: Doppler sensor
- 112: camera
- 141: SB control unit
- 142: behavior detection processing unit
- 143: notification processing unit
- 144: streaming processing unit
- 145: nurse-call processing unit
- 211: SV control unit
- 221: notification-destination information storage unit
- 311: TA control unit
- 312: TA processing unit
- 321: display-screen storage unit
- 411: notification-source address field
- 412: notification-event field
- 413: notification-destination address field
- 611: menu-bar region
- 612: main region
- 3121: display processing unit
- AP: access point
- IC1-IC5: icon
- MS: watching system
- NW: network
- Ob: object person
- RM: room
- SB: sensor box
- SP: stationary terminal
- ST: nurse station
- SV: management server
- TA: mobile terminal

## Claims

1. A watching system comprising:
a sensor device configured to input data from a person to be watched, the sensor device being configured to output a signal corresponding to the data;
a management server communicably connected to the sensor device through a network; and
a mobile terminal capable of wirelessly communicating with the management server through the network, the mobile terminal being configured to output a watching alarm, based on the signal transmitted from the sensor device through the management server,
wherein, when the mobile terminal transmits, to the management server through the network, login information including identifier information of the mobile terminal and information regarding a user operating the mobile terminal, the management server permits the mobile terminal to log in and the permission enables information to be downloaded from the management server to the mobile terminal,
when the mobile terminal transmits logout information to the management server, the management server permits the mobile terminal to log out and the permission disables the information from being downloaded from the management server to the mobile terminal,
an information saving unit that saves the login information used for the login of the mobile terminal, is further provided,
when the communication is unavailable between the mobile terminal and the management server before the transmission of the logout information after the mobile terminal transmits the login information, a re-login request is automatically transmitted from the mobile terminal and the management server retains the login of the mobile terminal on hold during a standby period, and
when the management server receives the re-login request during the standby period after recovery to the communication available between the mobile terminal and the management server, the management server resumes the login to the mobile terminal that has transmitted the re-login request, based on the login information read from the information saving unit.

2. The watching system according to claim 1, wherein the mobile terminal includes the information saving unit, the re-login request includes the login information read from the information saving unit, and in a case where, after the communication is unavailable with the management server when the mobile terminal goes out of a predetermined range in which the mobile terminal is capable of wirelessly communicating with the network, the mobile terminal returns within the predetermined range, the management server resumes the login to the mobile terminal, based on the login information included in the re-login request, when the management server receives the re-login request transmitted from the mobile terminal during the standby period.

3. The watching system according to claim 1, wherein the management server includes the information saving unit, the information saving unit stores a login list created, based on the login information, such that the identifier information of the mobile terminal in the login is associated with the information regarding the user operating the mobile terminal, in a case where, after the communication is unavailable with the management server when the mobile terminal goes out of a predetermined range in which the mobile terminal is capable of wirelessly communicating with the network, the mobile terminal returns within the predetermined range, when the management server receives the re-login request including at least the identifier information of the mobile terminal, transmitted from the mobile terminal during the standby period, the management server determines whether the mobile terminal that has transmitted the re-login request is included in the login list saved in the information saving unit, based on the transmitted re-login request, and resumes, when determining that the mobile terminal that has transmitted the re-login request is included, the login to the mobile terminal, based on the login information.

4. The watching system according to any of claims 1 to 3, wherein, when the mobile terminal goes out of the predetermined range before the transmission of the logout information after the transmission of the login information, the mobile terminal indicates information indicating that the communication is unavailable with the management server.

5. The watching system according to any of claims 1 to 4, wherein, when the mobile terminal has the communication unavailable with the management server, the re-login request is transmitted at least one time from the mobile terminal during a predetermined sending time, and the sending time is shorter than the standby time.

6. The watching system according to any of claims 1 to 5, wherein, when the management server does not receive the re-login request from the mobile terminal during the standby time, the management server performs logout processing to the mobile terminal.

7. A management server comprising:
a communication unit configured to communicate with a plurality of sensor devices and a plurality of mobile terminals through a network, the plurality of sensor devices each configured to input data from a person to be watched, each being configured to output a signal corresponding to the data, the plurality of mobile terminals being configured to output watching alarms, based on the signals transmitted from the sensor devices;
a storage unit configured to store a table including the plurality of sensor devices associated with the plurality of mobile terminals;
a notification processing unit configured to determine whether each of the plurality of mobile terminals is in a login, the notification processing unit being configured to issue, when receiving data from one sensor device of the plurality of sensor devices, notification to a mobile terminal in the login from mobile terminals associated with the one sensor device, the mobile terminals being stored in the storage unit; and
an information saving unit configured to save login information used for the login of the mobile terminal, wherein, when the communication unit receives the login information including identifier information of the mobile terminal and information regarding a user operating the mobile terminal, transmitted from the mobile terminal through the network, the mobile terminal is permitted to log in and the permission enables information to be downloaded to the mobile terminal,
when the communication unit receives logout information transmitted from the mobile terminal, the mobile terminal is permitted to log out and the permission disables the information from being downloaded to the mobile terminal,
when the communication is unavailable between the management server and the mobile terminal before the transmission of the logout information after the mobile terminal transmits the login information, a re-login request is automatically transmitted from the mobile terminal and the login of the mobile terminal is on hold during a standby period of the management server, and
when the communication unit receives the re-login request during the standby period after recovery to the communication available between the management server and the mobile terminal, the login is resumed to the mobile terminal that has transmitted the re-login request, based on the login information read from the information saving unit.
